(19) Europäisches Patentamt
European Patent Office
Office européen des brevets

(11) EP 4 041 707 B1

(12) EUROPEAN PATENT SPECIFICATION

(45) Date of publication and mention of the grant of the patent:
**19.02.2025 Bulletin 2025/08**

(21) Application number: **20804697.9**

(22) Date of filing: **06.10.2020**

(51) International Patent Classification (IPC):
***C07C 67/58*** *(2006.01)* ***C07C 69/675*** *(2006.01)*

(52) Cooperative Patent Classification (CPC):
(C-Sets available)
**C07C 67/58** (Cont.)

(86) International application number:
**PCT/IT2020/050246**

(87) International publication number:
**WO 2021/070209 (15.04.2021 Gazette 2021/15)**

(54) **METHOD TO PURIFY TRIS-(3-HYDROXYBUTYRATO)-GLYCERYL ESTER**

VERFAHREN ZUM REINIGEN VON TRIS-(3-HYDROXYBUTYRATO)-GLYCERYLESTER

PROCÉDÉ DE PURIFICATION D’ESTER TRIS-(3-HYDROXYBUTYRATO)-GLYCÉRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.10.2019 IT 201900018491**

(43) Date of publication of application:
**17.08.2022 Bulletin 2022/33**

(73) Proprietor: **Dr. Schär S.P.A.**
**39014 Postal (IT)**

(72) Inventors:
- **CERNE, Virna Lucia**
  **34011 AURISINA (IT)**
- **RAZZETTI, Gabriele**
  **20099 SESTO SAN GIOVANNI (IT)**
- **MANTEGAZZA, Simone**
  **20146 MILANO (IT)**
- **ROSSI, Roberto**
  **20156 MILANO (IT)**
- **CARBONI, Philippe**
  **47923 RIMINI (IT)**
- **SANTILLO, Niccolò**
  **53100 SIENA (IT)**
- **BRENNA, Davide**
  **20030 SENAGO (IT)**
- **ATTOLINO, Emanuele**
  **74019 PALAGIANO (IT)**

(74) Representative: **Petraz, Gilberto Luigi et al**
**GLP S.r.l.**
**Viale Europa Unita, 171**
**33100 Udine (IT)**

(56) References cited:
**US-A- 5 693 850**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 67/58, C07C 69/675**

## Description

FIELD OF THE INVENTION

The present invention is defined in the claims and concerns a method to prepare and purify glyceryl-tris-(3-hydroxybutyrate) ester, which is used in diets that require specific nutritional needs, such as for example the ketogenic diet.

BACKGROUND OF THE INVENTION

Glyceryl-tris-(3-hydroxybutyrate) ester, having the following structural formula (**I**):

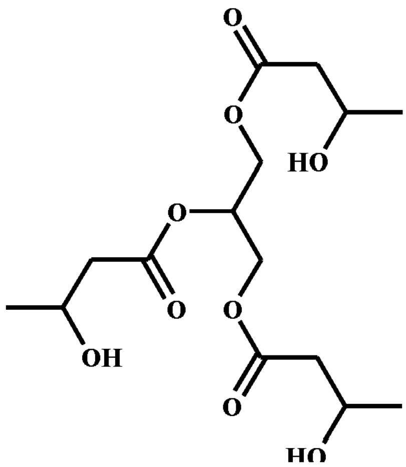

(I),

and in particular its enantiomer having all three stereogenic centers in configuration (R) of formula (**Ia**)

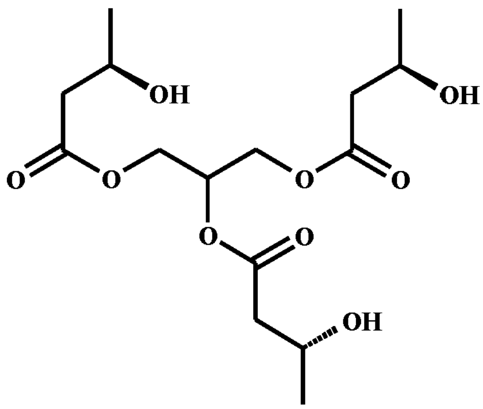

(Ia)

are used in the ketogenic diet (KD) which is a nutritional approach consisting of a high-fat content and low carbohydrate levels. A diet high in fat but low in carbohydrates leads to ketosis, where the body gains energy from fat in the form of ketone products. This type of diet has been used in the treatment of various diseases, for example in the treatment of infantile refractory epilepsy (E. van der Louw et al. Eur. J. Paediatr. Neurol. 2016 20, 798-809).

Patent application WO 95/09144 describes a method to obtain glyceryl-tris-(3-hydroxybutyrate) ester of formula (**I**) which provides a first esterification of the glycerin of formula (**II**) with tert-butyl acetoacetate of formula (**III**) and subsequent hydrogenation of the intermediate of formula (IV) in the presence of Raney nickel as catalyst.

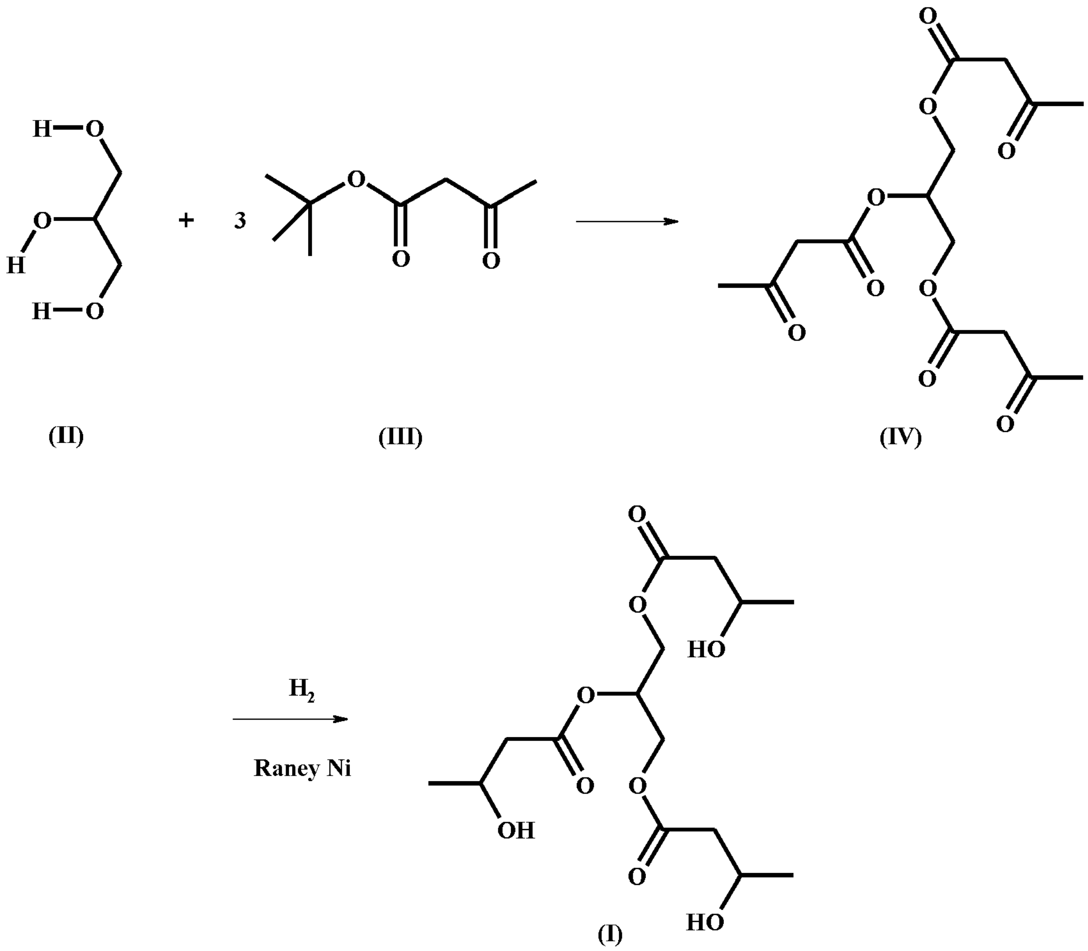


(II) (III) (IV)

(I)

While the condensation of the glycerin of formula (**II**) and the acetoacetate of formula (**III**) continues with a good yield, the hydrogenation in the presence of Raney nickel as catalyst requires a pressure of 1000 psi (6894,76 kPa), therefore about 70 bar, which is a very high pressure, especially in the perspective of production on an industrial scale. Furthermore, patent application WO 95/09144 describes the purification of the desired product by means of ion exchange resins necessary to remove the residual nickel in the product.

Patent application JP.03-083950 describes a method to obtain the optically active compound of formula (**Ia**) by means of a sequence of reactions which provide the initial protection of methyl 3-(R)-hydroxybutyrate of formula (**V**) as THP and subsequent hydrolysis to obtain the acid of formula (**VI**). The carboxylic acid is then activated by treatment with CDI and then esterified with the glycerin of formula (**II**) to obtain the protected intermediate of formula (**VII**). The deprotection reaction by the tetrahydropyranyl protective groups of the compound of formula (**VII**) in an acid environment finally leads to the optically active product of formula (**Ia**):

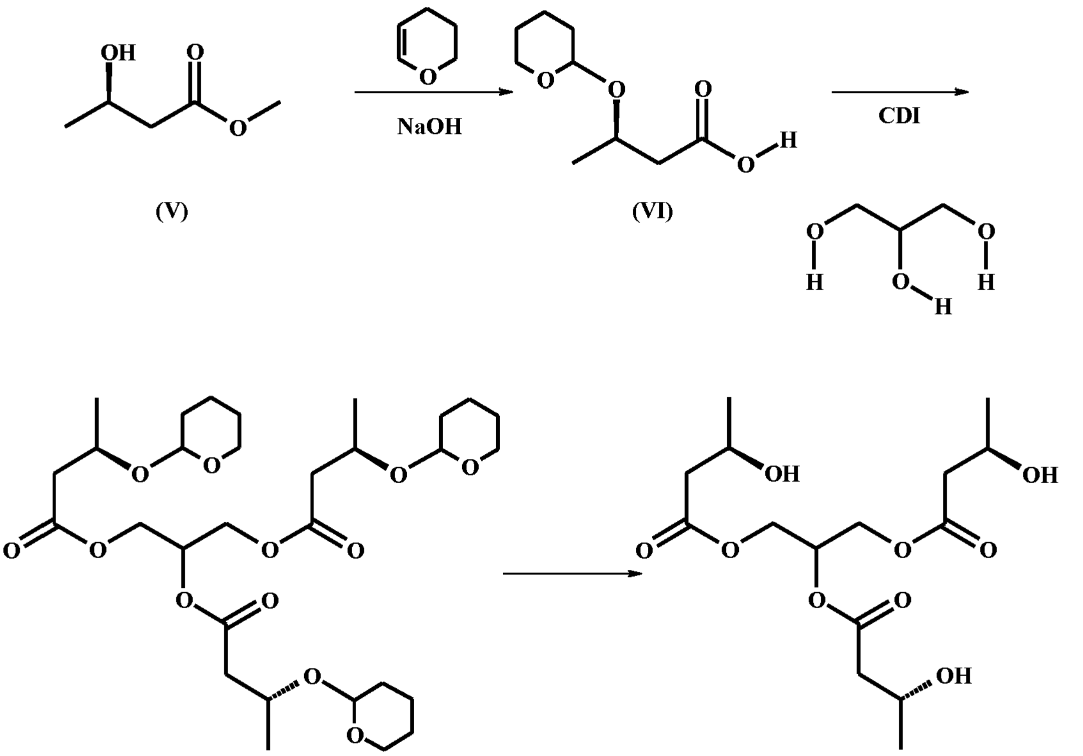


(V) (VI)

(VII) (Ia)

The method described in JP.03-083950 is a long method and leads to the desired product with low yields. Furthermore, the product of formula (**Ia**) is purified by chromatography to obtain the pure compound.

Both the compound of formula (**I**) and its optically active isomer (**Ia**) at room temperature present as oils, as in all cases known to the person of skill in the art for triglycerides of short-chain fatty acids. Since it is not possible to purify the compounds by crystallization, and since it is desired to avoid silica chromatography on an industrial scale, also the purification of a compound of formula (**I**) and formula (**Ia**) to obtain products having a suitable purity higher than 99% in A% HPLC is very complex, and so far it has not been reported in the literature that such a degree of purity has been achieved with different methods.

There is therefore a need for an alternative, simpler and more advantageous method to prepare glyceryl-tris-(3-hydroxybutyrate) ester of formula (**I**) and its optically active isomer having all three stereogenic centers with a configuration (R) of formula (**Ia**)**.** This new method should in particular provide a smaller number of synthetic steps, avoiding the extensive use of protective groups, and should improve the atom economy of the process. The method should also be economical, safe for humans and the environment, use mild reaction conditions and at the same time provide the desired compounds in high yields and high chemical and stereochemical purity.

SUMMARY OF THE INVENTION

The invention concerns a method to prepare a compound of formula (**I**)

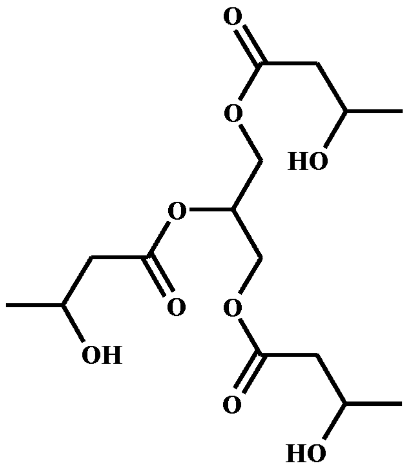

**(I),**

as a single enantiomer or as a mixture of isomers, comprising the hydrogenation reaction of a compound of formula (**IV**)

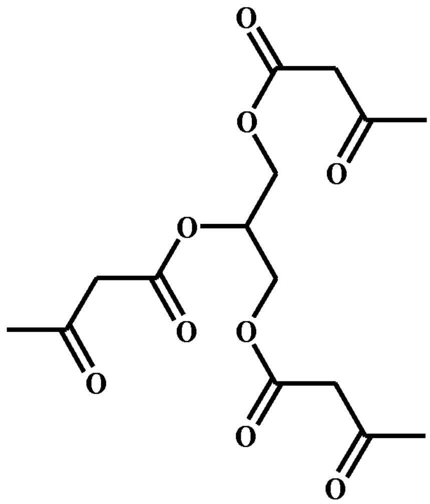

**(IV),**

in the presence of a ruthenium-based catalyst.

Another purpose of the present invention in a method to purify a compound of formula **(I)** as defined above comprising:

a. one or more washes of an aqueous solution of a compound of formula **(I)** and wherein the aqueous solution typically comprises NaCl from 0% to 5% w/w, with an organic solvent **S1,** as defined here;
b. increase of the concentration of NaCl of the aqueous solution of a compound of formula **(I)** as in point a. greater than 5% w/w;
c. one or more extractions of the aqueous solution of a compound of formula **(I)** as in point b. with s solvent **S2,** as defined here and
d. concentration of the solution of a compound of formula **(I)** in a solvent **S2** to obtain a compound of formula **(I)** as transparent oil.

The organic solvent **S1** is typically an organic solvent selected from a cyclic or acyclic ether or a non-polar aprotic solvent.

The organic solvent **S2** is typically an organic solvent selected from a polar aprotic solvent; a chlorinated solvent; an ester; or a linear or branched $C_3$-$C_7$ ketone.

DETAILED DESCRIPTION OF THE INVENTION

The invention concerns a method to prepare a compound of formula **(I)**

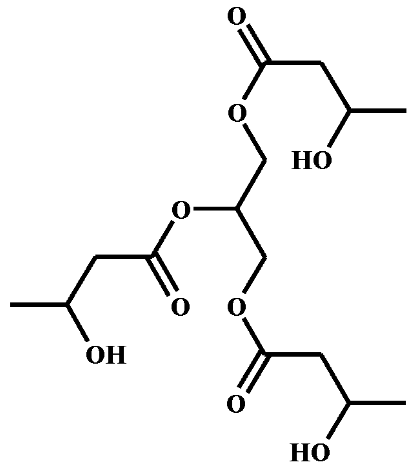

**(I),**

as a single enantiomer or as a mixture of isomers, comprising the hydrogenation reaction of a compound of formula **(IV)**

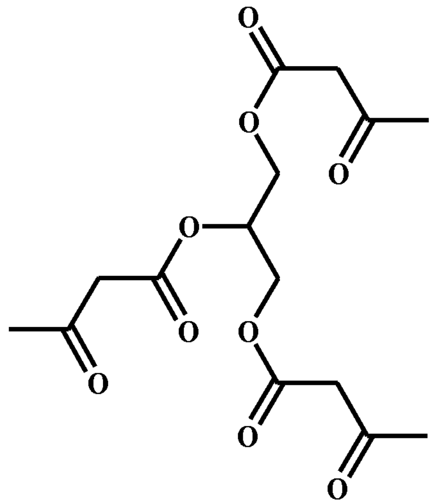

**(IV),**

in the presence of a ruthenium-based catalyst.

The hydrogenation of a compound of formula (IV) can be carried out by catalytic hydrogenation in the presence of a homogeneous or heterogeneous Ru-based metal catalyst.

When the metal catalyst is heterogeneous, it is preferably deposited on an inert support such as, for example, carbon, barium hydroxide, alumina, calcium carbonate; preferably carbon. The concentration of the metal on the support can vary between about 1 and 30%, preferably between about 5 and 20%.

The hydrogen pressure employed can vary between about 1 bar and about 50 bar, preferably between 2 bar and 40 bar, for example, at 3 bar, 4 bar, 5 bar, 6 bar, 7 bar, 8 bar, 9 bar, 10 bar, 15 bar, 20 bar, 25 bar, 30 bar or 35 bar.

The molar quantity of catalyst used, referred to the compound of formula **(IV),** is comprised between about 0.1 and 10%, preferably between about 0.5 and 5%.

The hydrogenation reaction can be carried out in the presence of an organic solvent selected, for example, from a polar aprotic solvent, typically dimethylformamide, dimethylacetamide, acetonitrile, dimethyl sulfoxide; a cyclic or acyclic ether, typically tetrahydrofuran or dioxane or methyl tert-butyl ether; a chlorinated solvent, typically dichloromethane; a

non-polar aprotic solvent, typically toluene or hexane; a polar protic solvent, such as a linear or branched $C_1$-$C_6$ alcohol, in particular methanol, ethanol, isopropanol or butanol; an ester, for example ethyl acetate, isopropyl acetate, butyl acetate; a carboxylic acid, for example acetic acid or propionic acid; or water; or mixtures of two or more of said solvents, preferably 2 or 3.

Preferably, the reaction can be carried out in a $C_1$-$C_6$ alcohol, for example ethanol or isopropanol, in an ester solvent, for example ethyl acetate, or in a mixture of an ester solvent, for example ethyl acetate, and water.

This hydrogenation reaction can be carried out at a temperature comprised between about 0°C and the reflux temperature of the solvent; preferably between about 25°C and the reflux temperature.

The hydrogenation reaction of a compound of formula **(IV)** can also be carried out by means of a hydrogen transfer reaction, using a homogeneous or heterogeneous metal catalyst, for example as defined above, and in the same molar quantity, and a hydrogen donor. The latter selected for example in the group comprising cyclohexene; cyclohexadiene; methylcyclohexene; limonene; dipentene; mentene; hydrazine; phosphinic acid or its derivatives, for example sodium hypophosphite; indoline; ascorbic acid; formic acid or its sodium or ammonium salts; and secondary alcohols, for example isopropanol.

The molar ratio between the hydrogen donor and the compound of formula **(IV)** can be comprised between about 1.5 and 50, preferably between about 1.5 and 10.

The hydrogen transfer reduction reaction can be carried out in the presence of an organic solvent, selected for example from one of the solvents mentioned above.

In a preferred aspect of the invention the catalyst is heterogeneous and is more preferably Ru/C.

In another preferred aspect of the invention the catalyst is homogeneous and is more preferably a Ruthenium complex with mono or diphosphine ligands well known in the chemistry of enantioselective hydrogenations, for example the homogeneous catalyst Ru((R)-BINAP)$Cl_2$.

In accordance with a preferred aspect of the invention, the hydrogenation of a compound of formula **(IV)** using the homogeneous catalyst Ru((R)-BINAP)$Cl_2$ allows to obtain a compound of formula **(Ia)**

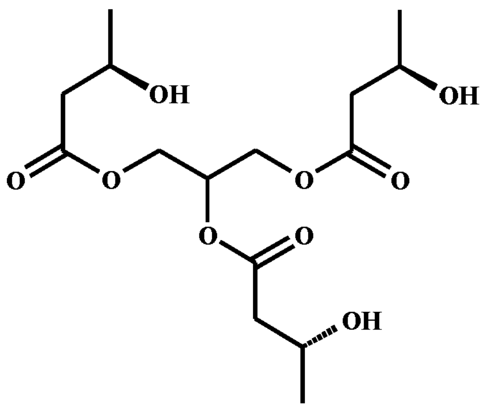

**(Ia),**

having all three stereogenic centers in configuration (R).

Therefore, according to another aspect, the invention provides an advantageous method to prepare a compound of formula **(Ia)**

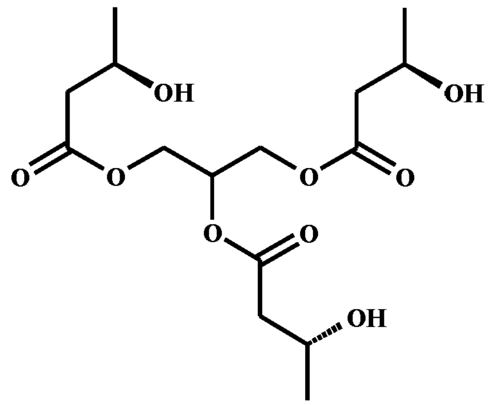

**(Ia),**

as defined here, comprising the hydrogenation reaction of a compound of formula **(IV)**

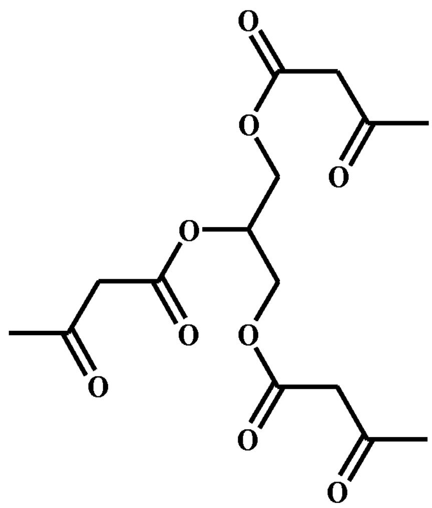

**(IV),**

in the presence of the homogeneous catalyst Ru((R)-BINAP)$Cl_2$.

A compound of formula **(IV)** is a known compound and can be obtained for example by esterification reaction of glycerin of formula **(II)**

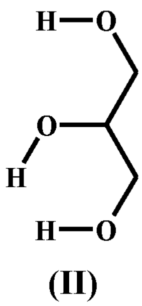

**(II)**

with tert-butyl acetoacetate of formula **(III)**

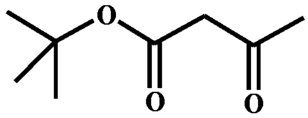

(III)

Compounds of formula **(II)** and formula **(III)** are commercially available.

At the end of the hydrogenation, both the glyceryl-tris(-3-hydroxybutyrate) ester of formula **(I)** as well as its enantiomer of formula **(Ia)** are obtained by evaporation of the solvent as liquids having a purity measured by means of HPLC always higher than 90%, but lower than 99%.

It has been surprisingly found that the glyceryl-tris(-3-hydroxybutyrate) ester of formula **(I)** and its enantiomer of formula **(Ia),** unlike common triglycerides which are lipophilic and insoluble in water such as the compound of formula **(IV),** have an amphiphilic nature which makes them soluble under specific experimental conditions both in water and also in its saline solutions as well as in organic solvent.

The present invention also concerns a method to purify a compound of formula **(I)** or of formula **(Ia)** comprising:

a. one or more washes of an aqueous solution of a compound of formula **(I)** or of formula **(Ia)** and wherein the aqueous solution comprises NaCl from 0% to 5% w/w, preferably from 1% to 5% w/w, with an organic solvent S1, as defined here;

b. increase of the concentration of NaCl of the aqueous solution of a compound of formula **(I)** or **(Ia)** as in point a. greater than 5% w/w, preferably at least 10% w/w, more preferably at least 15% w/w;

c. one or more extractions of the aqueous solution of a compound of formula **(I)** or of formula **(Ia)** as in point b. with an organic solvent **S2,** as defined here; and

d. concentration of the solution of a compound of formula **(I)** or formula **(Ia)** in an organic solvent **S2** in order to obtain a compound of formula **(I)** or of formula **(Ia)** as a transparent oil, typically with a purity measured by means of HPLC greater than 99%.

A solvent **S1** is an organic solvent selected, for example, from a cyclic or acyclic ether, typically diethyl ether or methyl tert-butyl ether, typically methyl tert-butyl ether; a non-polar aprotic solvent, typically toluene.

A solvent **S2** is an organic solvent selected, for example, from a polar aprotic solvent, typically acetonitrile; a chlorinated solvent, typically dichloromethane; an ester, for example ethyl acetate, isopropyl acetate, butyl acetate, preferably ethyl acetate; a linear or branched $C_3$-$C_7$ ketone, for example, methyl ethyl ketone, methyl isobutyl ketone.

The washing with the solvent **S1** or the extraction with the solvent **S2** of a compound of formula **(I)** or of formula **(Ia)** can be carried out at a temperature comprised between about 0°C and about 60°C; preferably between about 25°C and about 60°C, for example at 30°C, at 35°C, at 40°C, at 45°C, at 50°C or at 55°C.

The solution of a compound of formula **(I)** or of formula **(Ia)** in a solvent **S2** can be anhydrated by drying. Drying can be carried out by means of anhydration with a dehydrating agent, for example sodium sulfate ($Na_2SO_4$), magnesium sulfate ($MgSO_4$) or anhydrous calcium chloride ($CaCl_2$), preferably sodium sulfate ($Na_2SO_4$).

A compound of formula **(I)** or formula **(Ia)** as a transparent oil with a purity measured by means of HPLC greater than 99% has never been obtained without the aid of chromatographic purification techniques.

The present invention also concerns a method to purify a compound of formula **(I)** or of formula **(Ia)** with a purity measured by means of HPLC greater than 99% without the aid of purification techniques of the chromatographic type, for example without proceeding with chromatographic purification on ion exchange resin or normal or inverse stationary phases.

The extraction in an organic solvent of a compound of formula **(I)** or of formula **(Ia)** from an aqueous solution in accordance with point c. of the purification process also allowed to bring down the content of all heavy metals and of ruthenium in particular in a compound of formula **(I)** or formula **(Ia)** well below the limits provided by the ICH guidelines, and which previously had only been possible by means of chromatographic purification on ion exchange resin.

Therefore, the present invention also concerns a compound of formula **(I)** or formula **(Ia),** obtained in accordance with the process and purification method object of the present invention, having a content of heavy metals lower than 0.5 ppm.

The following examples further illustrate the invention:

**Example 1 - Synthesis of glycerol tris-acetoacetate of formula (IV)**

Glycerin (150 g, 1.69 mol), tert-butyl acetoacetate (1350 g, 8.55 mol) are added to a 3000 ml flask in inert atmosphere and the mixture is heated at 95-100°C for 2.5 hours. The reaction mixture is then concentrated at a reduced

pressure of 200-250 mbar and at an internal temperature of 80-90°C, integrating toluene in portions to the reaction mixture for a total of 1.5 1. The end of distillation residue is then cooled at 0-10°C and diluted with isopropanol cooled at -10°C (2.5 1), the phases are separated, discarding the alcoholic phase and obtaining a crude oil (620 g) which is repeatedly washed with cold isopropanol until a product (400 g) is obtained with an HPLC purity (200 nm) in A% of 98.6% with a glycerol bis-acetoacetate content lower than 0.3% and a yield of 70%.

$^1$H-NMR ($CDCl_3$, 300 MHz) δ: 11.8* (s, 1H); 5.34 (m, 1H); 4.99* (s, 1H); 4.32 (m, 4H); 3.49 (s, 6H); 2.25 (s, 9H); 1.96* (s, 3H). *Keto-enol isomerism.
HPLC-MS: 345 (M/z+1)

**Example 2 - Synthesis of glyceryl-tris(-3-hydroxybutyrate) ester of formula (I)**

A solution of glycerol tris-acetoacetate of formula **(IV)** (100 g, 0.29 mol) in ethyl acetate (500 ml) and the ruthenium on carbon at 5% with water content approximately 50% (30 g) are loaded into a 1000 ml autoclave at room temperature. The autoclave is inerted with nitrogen and after vacuuming it, it is pressurized with hydrogen at 4.5-5 bar and 1000 rpm of stirring for 6-8 hours. The reaction is monitored by means of HPLC analysis and when the reaction is complete the reactor is inerted and the catalyst is filtered on perlite, washing with ethyl acetate (100 ml). The solution is concentrated to residue at reduced pressure and at a temperature of 30-35°C. The crude is dissolved in water (350 ml), treated with decoloring carbon (2.5 g), left under stirring for 2 hours then filtered on perlite and washed with water (150 ml). Sodium chloride (25 g) and methyl tert-butyl ether (140 ml) are added to the aqueous phase. The two phases are left under vigorous stirring for 30 minutes at a temperature of 45-50°C and are separated, discarding the organic phase. More sodium chloride (50 g) and ethyl acetate (400 ml) are added to the aqueous phase. The phases are left under stirring at 45-50°C for 30 minutes and the phases are separated. The aqueous phase is extracted with further ethyl acetate and the organic phases are reunited, anhydrated on sodium sulfate and concentrated to residue at a reduced pressure at a temperature of 30-35°C. The washing and extraction procedure is repeated 3 times obtaining 72 g of the compound of formula **(I)** as a colorless oil with HPLC purity (200 nm) at 99.3% in A% and a yield of 71%.

$^1$H-NMR ($CDCl_3$, 300 MHz) δ: 5.32 (m, 1H); 4.44-4.10 (m, 7H); 2.76 (s, 3H); 2.52-2.35 (m, 6H); 1.21 (d, 9H).
HPLC-MS: 351 (M/z+1)

**Example 3 - Synthesis of glyceryl-tris-(3- R)-hydroxybutyrate) ester of formula (Ia)**

A solution of glycerol tris-acetoacetate (150 g, 0.43 mol) and the catalyst Ru((R)-BINAP)$Cl_2$ (0.69 g, 0.87 mmol) in ethanol (500 ml) are loaded into a 1000 ml autoclave at room temperature. The autoclave is inerted with nitrogen, heated at 40-45°C and after vacuuming it is pressurized with hydrogen at 35 bar and 1600 rpm for 6-8 hours. The disappearance of the starting product is verified by means of HPLC analysis, the autoclave is unloaded, and the solution is filtered on a perlite and carbon panel. The solution filtered to residue is concentrated at a temperature of 45-50°C and at a reduced pressure. The product is dissolved in water (500 ml) and decoloring carbon (3.75 g) is added, leaving it under vigorous stirring for 2 hours at room temperature. The crude solution is filtered on a perlite panel washing with water (225 ml) and the aqueous solution is used in the purification step.

$^1$H-NMR ($CDCl_3$, 300 MHz) δ: 5.32 (m, 1H); 4.44-4.10 (m, 7H); 2.76 (s, 3H); 2.52-2.35 (m, 6H); 1.21 (d, 9H).
HPLC-MS: 351 (M/z+1).

**Example 4 - Purification of glyceryl-tris-(3-(R)-hydroxybutyrate) ester of formula (Ia)**

Sodium chloride (36 g) is added to the solution obtained in Example 3, it is heated at the temperature of 45-50°C and the aqueous phase is washed with toluene (2x225 ml) and subsequently with methyl tert-butyl ether (2x200 ml). Sodium chloride (180 g) is added to the aqueous solution and the product is extracted with ethyl acetate (2x200 ml), the reunited organic phases are anhydrated with sodium sulphate, filtered on perlite and concentrated to residue at the temperature of 45-50°C and at reduced pressure. 120 g of glyceryl-tris-(3-(R)-hydroxybutyrate) ester of formula **(Ia)** are obtained, with an HPLC purity (200 nm) of 99.1% in A% and a yield of 79%. Rotary optical power

$[\alpha]_D^{25}$(C=1.15 Methanol) = -22.0°.

## Claims

1. Method to purify a compound of formula **(I)**

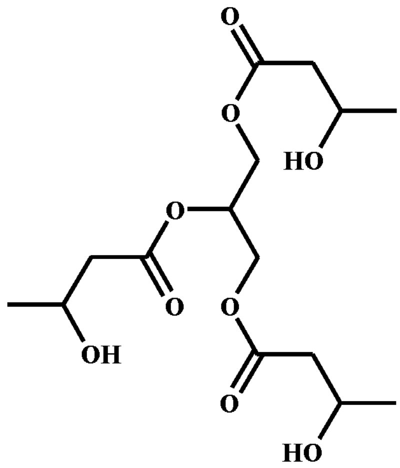

**(I),**

as a single enantiomer or as a mixture of isomers, comprising:

a. one or more washes of an aqueous solution of a compound of formula **(I)** and wherein the aqueous solution comprises NaCl from 0% to 5% w/w, with an organic solvent **S1;**
b. increase of the concentration of NaCl of the aqueous solution of a compound of formula **(I)** as in point a. greater than 5% w/w;
c. one or more extractions of the aqueous solution of a compound of formula **(I)** as in point b. with an organic solvent **S2,** and
d. concentration of the solution of a compound of formula **(I)** in an organic solvent **S2** to obtain a compound of formula **(I),**

and wherein the solvent **S1** is an organic solvent selected from a cyclic or acyclic ether or a non-polar aprotic solvent, and wherein the solvent **S2** is an organic solvent selected from a polar aprotic solvent; a chlorinated solvent; an ester; or a linear or branched $C_3$-$C_7$ ketone.

2. The method as in claim 1, wherein the compound of formula **(I)** is a compound of formula **(Ia),**

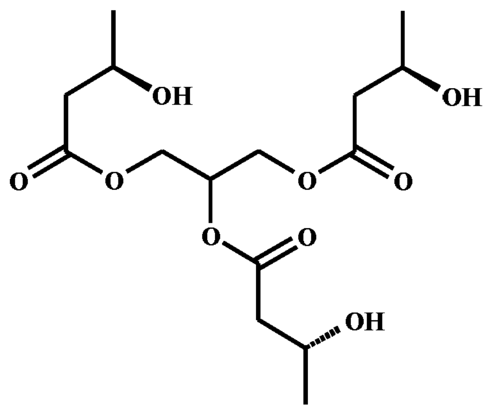

**(Ia).**

3. The method as in claims 1 or 2, wherein the aqueous solution as in point a. comprises NaCl from 1% to 5% w/w.

4. The method as in claims from 1 to 3, wherein the aqueous solution as in point b. comprises NaCl at least 10% w/w.

5. The method as in claim 4, wherein the aqueous solution as in point b. comprises NaCl at least 15% w/w.

6. The method as in claims from 1 to 5, wherein the organic solvent **S1** is diethyl ether, methyl tert-butyl ether, or toluene.

7. The method as in claims from 1 to 6, wherein the organic solvent **S2** is acetonitrile, dichloromethane, ethyl acetate, isopropyl acetate, butyl acetate, methyl ethyl ketone, or methyl isobutyl ketone.

8. The method as in claims from 1 to 7, wherein none of the steps comprises a purification by means of chromatography.

9. The method as in claims from 1 to 8, wherein the method includes obtaining the compound of formula **(I),** wherein said compound of formula **(I)** is obtained by means of a method comprising the hydrogenation reaction of a compound of formula **(IV)**

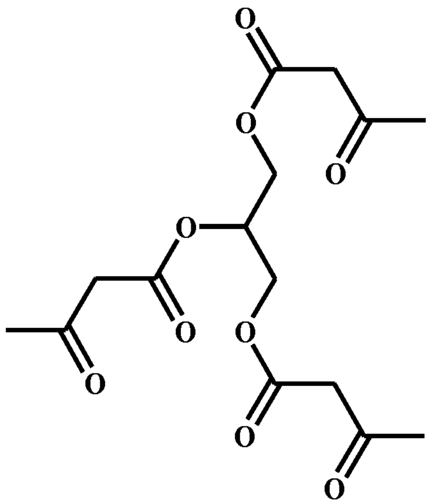

**(IV),**

in the presence of a ruthenium-based catalyst.

10. The method as in claims from 1 to 9, wherein the compound of formula **(I)** as defined in claim 1 or the compound of formula **(Ia)** as defined in claim 2 have a content of heavy metals less than 0.5 ppm.

**Patentansprüche**

1. Verfahren, um eine Verbindung der Formel **(I)** zu reinigen,

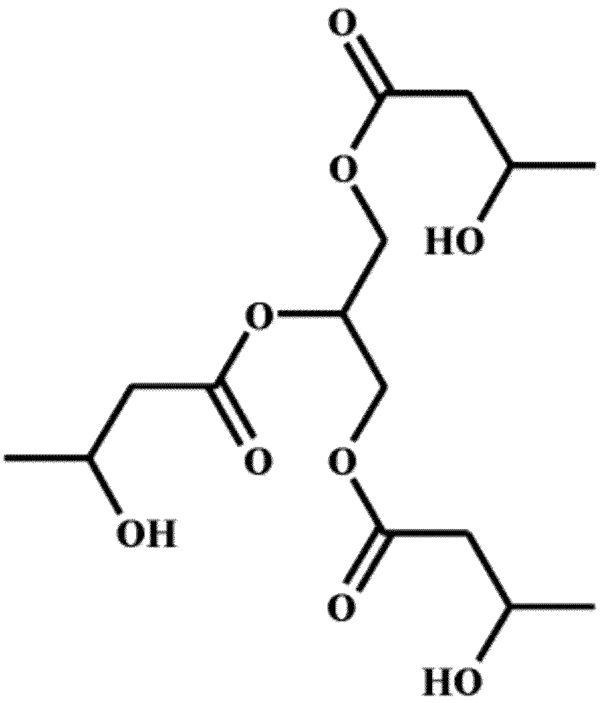

(I),

als ein einzelnes Enantiomer oder eine Mischung von Isomeren, umfassend:

a. eine oder mehr Waschungen einer wässrigen Lösung einer Verbindung der Formel **(I)** und wobei die wässrige Lösung NaCl von 0% bis 5% m/m, mit einem organischen Lösungsmittel **S1,** umfasst;
b. Steigerung der Konzentration von NaCl der wässrigen Lösung einer Verbindung der Formel **(I)** wie in Punkt a. größer als 5% m/m;
c. eine oder mehr Extraktionen der wässrigen Lösung einer Verbindung der Formel **(I),** wie in Punkt b. mit einem organischen Lösungsmittel **S2,** und
d. Konzentration der Lösung einer Verbindung der Formel **(I)** in einem organischen Lösungsmittel **S2,** um eine Verbindung der Formel **(I)** zu erhalten,

und wobei das Lösungsmittel **S1** ein organisches Lösungsmittel ist, ausgewählt aus einem zyklischen oder azyklischen Ether oder einem nicht-polaren aprotischem Lösungsmittel, und wobei das Lösungsmittel **S2** ein organisches Lösungsmittel ist, ausgewählt aus einem polaren aprotischem Lösungsmittel; einem chlorierten Lösungsmittel; einem Ester; oder ein einem linearen oder verzweigten $C_3$-$C_7$ Keton.

**2.** Verfahren wie in Anspruch 1, wobei die Verbindung der Formel **(I)** eine Verbindung der Formel **(Ia)** ist,

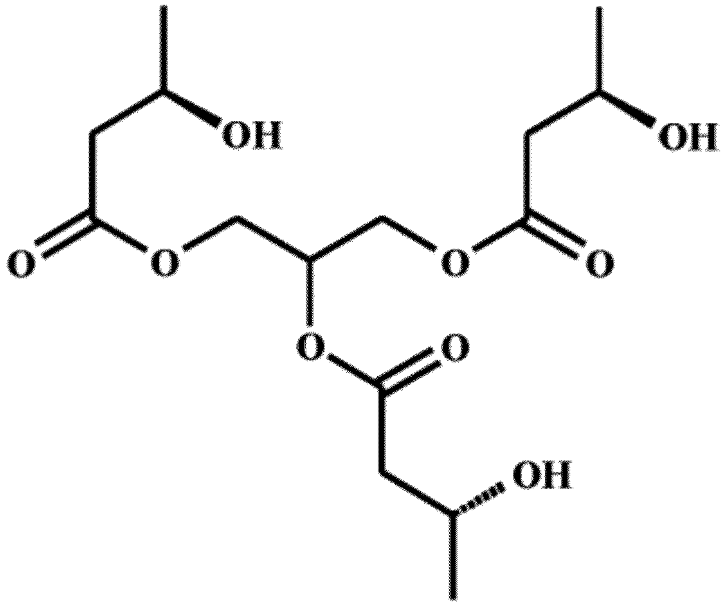

(Ia).

3. Verfahren wie in Ansprüchen von 1 oder 2, wobei die wässrige Lösung wie in Punkt a. NaCl von 1% bis 5% m/m umfasst.

4. Verfahren wie in Ansprüchen von 1 bis 3, wobei die wässrige Lösung wie in Punkt b. NaCl mindestens 10% m/m umfasst.

5. Verfahren wie in Anspruch 4, wobei die wässrige Lösung wie in Punkt b. NaCl mindestens 15% m/m umfasst.

6. Verfahren wie in Ansprüchen von 1 bis 5, wobei das organische Lösungsmittel **S1** Diethylether, Methyl-tert-butylether oder Toluol ist.

7. Verfahren wie in Ansprüchen von 1 bis 6, wobei das organische Lösungsmittel **S2** Acetonitril, Dichlormethan, Ethylacetat, Isopropylacetat, Butylacetat, Methylethylketon, oder Methylisobutylketon ist.

8. Verfahren wie in Ansprüchen von 1 bis 7, wobei keiner der Schritte eine Reinigung mittels Chromatographie umfasst.

9. Verfahren wie in Ansprüchen von 1 bis 8, wobei das Verfahren den Erhalt der Verbindung der Formel **(I)** beinhaltet, wobei die Verbindung der Formel **(I)** mittels eines Verfahrens, das die Hydrierreaktion einer Verbindung der Formel **(IV)**

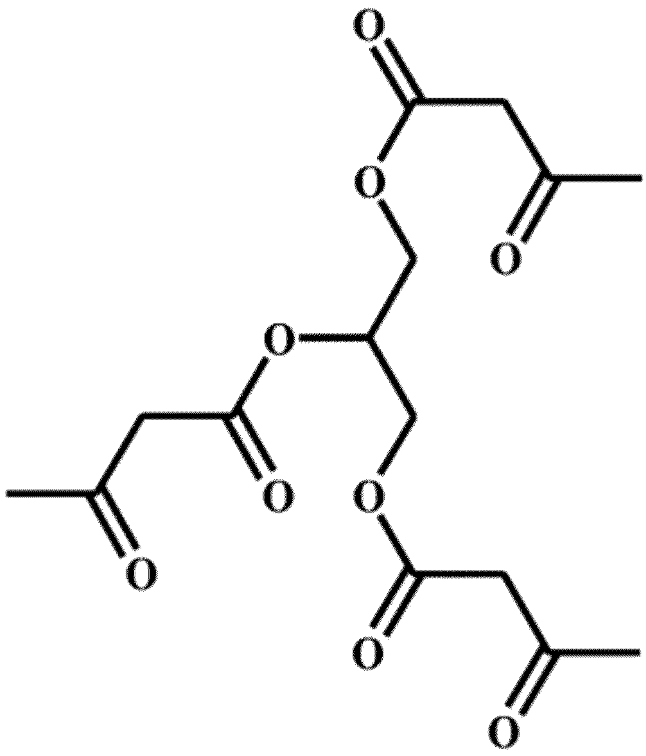

(IV),

in Anwesenheit eines Ruthenium-basierten Katalysators umfasst, erhalten wird.

**10.** Verfahren wie in Ansprüchen von 1 bis 9, wobei die Verbindung der Formel **(I),** wie in Anspruch 1 definiert, oder die Verbindung der Formel **(Ia),** wie in Anspruch 2 definiert, einen Anteil von Schwermetallen von weniger als 0,5 ppm aufweist.

## Revendications

**1.** Méthode de purification d'un composé de formule **(I)**

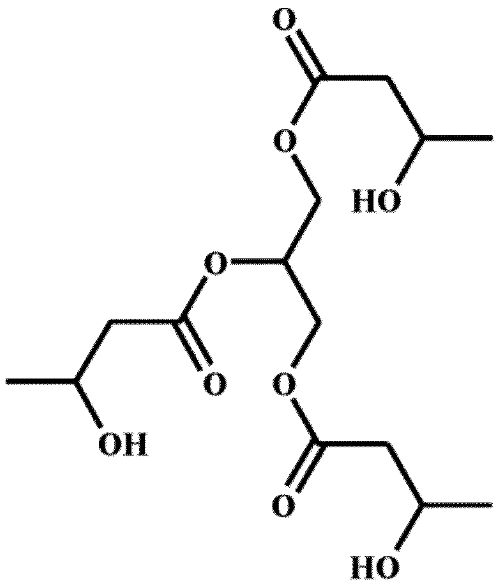

(I),

sous forme d'un énantiomère unique ou d'un mélange d'isomères, comprenant :

a) un ou plusieurs lavages d'une solution aqueuse d'un composé de formule (I) et dans laquelle la solution aqueuse comprend du NaCl de 0 % à 5 % p/p, avec un solvant organique **S1** ;
b) augmentation de la concentration en NaCl de la solution aqueuse d'un composé de la formule **(I)** selon le point a. supérieure à 5 % p/p ;
c) une ou plusieurs extractions de la solution aqueuse d'un composé de formule **(I)** selon le point b. avec un solvant organique **S2,** et
d) concentration de la solution d'un composé de formule **(I)** dans un solvant organique **S2** pour obtenir un composé de formule **(I),**

et dans laquelle le solvant **S1** est un solvant organique choisi parmi un éther cyclique ou acyclique ou un solvant aprotique non polaire, et dans laquelle le solvant **S2** est un solvant organique choisi parmi un solvant aprotique polaire, un solvant chloré, un ester ou une cétone en $C_3$-$C_7$ linéaire ou ramifiée.

**2.** Méthode selon la revendication 1, dans laquelle le composé de formule **(I)** est un composé de formule **(Ia),**

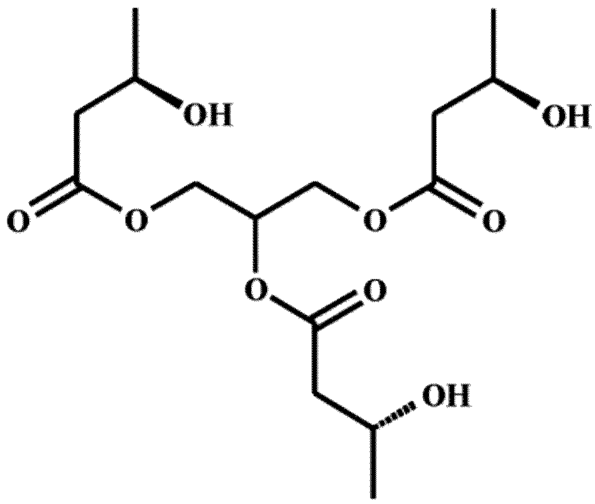

(Ia).

3. Méthode selon les revendications 1 ou 2, dans laquelle la solution aqueuse selon le point a. comprend du NaCl de 1 % à 5 % p/p.

4. Méthode selon les revendications 1 à 3, dans laquelle la solution aqueuse selon le point b. comprend du NaCl à raison d'au moins 10 % p/p.

5. Méthode selon la revendication 4, dans laquelle la solution aqueuse selon le point b. comprend du NaCl à raison d'au moins 15 % p/p.

6. Méthode selon les revendications 1 à 5, dans laquelle le solvant organique S1 est l'éther de diéthyle, l'éther de méthyle et de tert-butyle ou le toluène.

7. Méthode selon les revendications 1 à 6, dans laquelle le solvant organique **S2** est l'acétonitrile, le dichlorométhane, l'acétate d'éthyle, l'acétate d'isopropyle, l'acétate de butyle, la méthyléthylcétone ou la méthylisobutylcétone.

8. Méthode selon les revendications 1 à 7, dans laquelle aucune des étapes ne comprend une purification par chromatographie.

9. Méthode selon les revendications 1 à 8, dans laquelle la méthode comprend l'obtention du composé de formule **(I),** dans laquelle ledit composé de formule **(I)** est obtenu au moyen d'une méthode comprenant la réaction d'hydrogénation d'un composé de formule **(IV)**

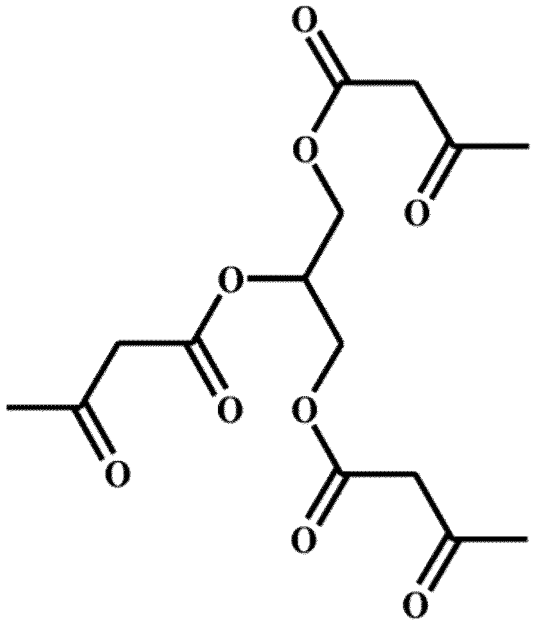

(IV),

en présence d'un catalyseur à base de ruthénium.

**10.** Méthode selon les revendications 1 à 9, dans laquelle le composé de formule (I) tel que que défini dans la revendication 1 ou le composé de formule **(Ia)** défini dans la revendication 2 ont une teneur en métaux lourds inférieure à 0,5 ppm.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9509144 A
- JP 3083950 A


### Non-patent literature cited in the description


- **E. VAN DER LOUW et al.** *Eur. J. Paediatr. Neurol.*, 2016, vol. 20, 798-809